# EUROPÄISCHE PATENTANMELDUNG

(11) **EP 1 371 985 A2**
(43) Veröffentlichungstag der Anmeldung: **17.12.2003**
(21) Anmeldenummer: 03012458.0
(22) Anmeldetag: 30.05.2003
(51) Int. Cl.: G01N 33/569, C12Q 1/68

(54) **Mittel und Verfahren zum Anreichern und Nachweisen von Mikroorganismen**

(30) Priorität: 29.05.2002 DE 10224338
(71) Anmelder: Gerlach, Gerald-F., 30449 Hannover (DE)
(72) Erfinder: Gerlach, Gerald-F., 30449 Hannover (DE)
(74) Vertreter: Eisenführ, Speiser & Partner

(57) **Zusammenfassung**

Die vorliegende Erfindung betrifft Mittel und Verfahren zum Anreichern und Nachweisen von Mikroorganismen. Die Erfindung betrifft insbesondere Anreicherungsverfahren und Immobilisierungsverfahren zum Immobilisieren von Mikroorganismen, sowie dazu vorteilhafte Peptide, Reagenzien und Kits. Die Erfindung betrifft femer Nachweisverfahren zum Nachweisen von Mikroorganismen, sowie dazu vorteilhafte Reagenzien und Kits. Ferner betrifft die Erfindung Verfahren zum Herstellen von Peptiden, die an Mikroorganismen binden können, sowie dazu besonders geeignete Puffer und Nucleinsäuren. Die Erfindung betrifft insbesondere auch Anreicherungs- und Nachweisverfahren zum Anreichern und Nachweisen von *Mycobacterium paratuberculosis* sowie in diesem Zusammenhang vorteilhafte Peptide.

SEQUENCE LISTING

<110>  Gerlach, Gerald F

<120>  Mittel und Verfahren zum Anreichern und Nachweisen von
Mikroorganismen

<130>  GA 597-02EP

<160>  95

<170>  PatentIn version 3.1

<210>  1
<211>  12
<212>  PRT
<213>  Artificial Sequence

<220>
<223>  generated by phage panning
<400>  1

Ser Thr Val Pro Arg Met Pro Leu Ser Pro Pro Asn
1               5                   10

<210>  2
<211>  12
<212>  PRT
<213>  Artificial Sequence

<220>
<223>  generated by phage panning
<400>  2

Phe Met Asn Pro Leu His Pro Arg Val Leu Arg Pro

1               5                   10

<210>  3

<211>  12

<212>  PRT

<213>  Artificial Sequence

<220>

<223>  generated by phage panning

<400>  3

Asn Tyr Val Ile His Asp Val Pro Arg His Pro Ala
1               5                   10

<210>  4

<211>  12

<212>  PRT

<213>  Artificial Sequence

<220>

<223>  generated by phage panning

<400>  4

Val Asn Met Gly Asn Tyr Arg Gln Gln Gln Pro Leu
1               5                   10

<210>  5

<211>  12

<212>  PRT

<213>  Artificial Sequence

<220>

<223>  generated by phage panning

<400>  5

Ser Met Leu Asp Leu Phe Pro Arg Ala Ala Ser Tyr
1               5                   10

<210>  6

<211>  12

<212>  PRT

<213>  Artificial Sequence

<220>

<223>  generated by phage panning

<400>  6

Ser Val Tyr Asn Val Arg Pro Ser Ser Leu Ser Ala
1               5                   10

<210>  7

<211>  12

<212>  PRT

<213>  Artificial Sequence

<220>

<223>  generated by phage panning

<400>  7

Thr Val Met Ala Ser Pro Thr Met Lys Ser Asn Ser
1               5                   10

<210>  8

<211>  12

<212>  PRT

<213>  Artificial Sequence

<220>

<223>  generated by phage panning

<400>  8

Val Asn Met Gly Asn Tyr Arg Gln Gln Gln Pro Gln
1               5                   10

<210>  9

<211>  12

<212>  PRT

<213>  Artificial Sequence



<220>

<223>  generated by phage panning

<400>  9

Lys Phe Pro Gly Pro Asn Cys Cys His Ala Leu Pro
1               5                   10


<210>  10

<211>  20

<212>  DNA

<213>  Artificial Sequence



<220>

<223>  1st PCR primer

<400>  10
gtatcaggcc gtgatggcgg                                                   20


<210>  11

<211>  20

<212>  DNA

<213>  Artificial Sequence



<220>

<223>  2nd PCR primer

<400>  11
ccgcaccagc gctcgataca                                                   20


<210>  12

<211>  36

<212>  DNA

<213>  Artificial Sequence

<220>

<223>  generated by phage panning

<400>  12
tctactgtgc cgcgtatgcc gctttctccg cctaat                                36

<210>  13

<211>  36

<212>  DNA

<213>  Artificial Sequence

<220>

<223>  generated by phage panning

<400>  13
tttatgaatc ctcttcatcc gagggtgttg cggcct                                36

<210>  14

<211>  36

<212>  DNA

<213>  Artificial Sequence

<220>

<223>  generated by phage panning

<400>  14
aattatgtga atcatgatgt gccgcgtcat cctgcg                                36

<210>  15

<211>  36

<212>  DNA

<213>  Artificial Sequence

<220>

<223>  generated by phage panning

<400>  15
gtgaatatgg ggaattatag gcagcagcag ccgctg                                36

<210> 16

<211> 36

<212> DNA

<213> Artificial Sequence

<220>

<223> generated by phage panning

<400> 16
agtatgctgg atctttttcc gcgtgctgct tcttat 36

<210> 17

<211> 36

<212> DNA

<213> Artificial Sequence

<220>

<223> generated by phage panning

<400> 17
tctgtgtata atgttcggcc ttcttcgctg tctgct 36

<210> 18

<211> 36

<212> DNA

<213> Artificial Sequence

<220>

<223> generated by phage panning

<400> 18
actgtgatgg cttctccgac gatgaagagt aattct 36

<210> 19

<211> 36

<212> DNA

<213> Artificial Sequence

<220>

<223>  generated by phage panning

<400>  19
gtgaatatgg ggaattatag gcagcagcag ccccag                                36


<210>  20

<211>  36

<212>  DNA

<213>  Artificial Sequence



<220>

<223>  generated by phage panning

<400>  20
aagtttccgg gtcctaattg ttgtcatgct ctgccg                                36


<210>  21

<211>  36

<212>  DNA

<213>  Artificial Sequence



<220>

<223>  degenerated sequence coding for peptide #1

<220>

<221>  misc_feature

<222>  (3)..(36)

<223>  n: a or c or g or t
       y: c or t


<400>  21
tcnacngtnc cncgnatgcc nctntcnccn ccnaay                                36


<210>  22

<211>  36

<212>  DNA

<213>  Artificial Sequence

<220>

<223>  degenerated sequence coding for peptide #2

<220>

<221>  misc_feature

<222>  (3)..(36)

<223>  n: a or c or g or t
       y: c or t

<400>  22
ttyatgaayc cnctncaycc ncgngtnctn cgnccn                                36

<210>  23

<211>  36

<212>  DNA

<213>  Artificial Sequence

<220>

<223>  degenerated sequence coding for peptide #3

<220>

<221>  misc_feature

<222>  (3)..(36)

<223>  n: a ro c or g or t
       h: a or c or t
       y: c or t

<400>  23
aaytaygtna thcaygaygt nccncgncay ccngcn                                36

<210>  24

<211>  36

<212>  DNA

<213>  Artificial Sequence

<220>

<223>  degenerated sequence coding for peptide #4

<220>

<221>  misc_feature

<222>  (3)..(36)

<223>  n: a or c or g or t
       r: a or g
       y: c or t

<400>  24
gtnaayatgg gnaaytaycg ncarcarcar ccnctn                                36

<210>  25

<211>  36

<212>  DNA

<213>  Artificial Sequence

<220>

<223>  degenerated sequence coding for peptide #5

<220>

<221>  misc_feature

<222>  (3)..(36)

<223>  n: a or c or g or t
       y: c or t

<400>  25
tcnatgctng ayctnttycc ncgngcngcn tcntay                                36

<210>  26

<211>  36

<212>  DNA

<213>  Artificial Sequence

<220>

<223>  degenerated sequence coding for peptide #6

<220>

<221>  misc_feature

<222>  (3)..(36)

<223>  n: a or c or g or t
       y: c or t

<400>  26
tcngtntaya aygtncgncc ntcntcnctn tcngcn                                36

<210>  27

<211>  36

<212>  DNA

<213>  Artificial Sequence

<220>

<223>  degenerated sequence coding for peptide #7

<220>

<221>  misc_feature

<222>  (3)..(36)

<223>  n: a or c or g or t
       r: a or g
       y: c or t

<400>  27
acngtnatgg cntcnccnac natgaartcn aaytcn                                36

<210>  28

<211>  36

<212>  DNA

<213>  Artificial Sequence

<220>

<223>  degenerated sequence coding for peptide #8

<220>

<221>  misc_feature

<222>  (3)..(36)

<223>  n: a or c or g or t
       r: a or g
       y: c or t

<400>  28
gtnaayatgg gnaaytaycg ncarcarcar ccncar                                36

<210>  29

<211>  36

<212>  DNA

<213>  Artificial Sequence

<220>

<223>  degenerated sequence coding for peptide #9

<220>

<221>  misc_feature

<222>  (3)..(36)

<223>  n: a or c or g or t
       r: a or g
       y: c or t

<400>  29
aarttyccng gnccnaaytg ytgycaygcn ctnccn                                36

<210>  30

<211>  36

<212>  DNA

<213>  Artificial Sequence

<220>

<223>  fMptD1; generated by phage panning; frequency of occurrence: 9

<400>  30
gggaagaatc atcatcatca gcatcatagg cctcag                                36

<210>  31

<211>  36

<212>  DNA

<213>  Artificial Sequence

<220>

<223>  fMptD2; generated by phage panning; frequency of occurrence: 2

<400>  31
tggccttcgt ggacgtctac tagtgggctt acgaag                                36

<210>  32

<211>  36

<212>  DNA

<213>  Artificial Sequence

<220>

<223>  fMptD3; generated by phage panning; frequency of occurrence: 2

<400>  32
gagattctgt ggtcgcgtgt tccggctacg actagg                                36

<210>  33

<211>  36

<212>  DNA

<213>  Artificial Sequence

<220>

<223>  fMptD4; generated by phage panning; frequency of occurrence: 1

<400>  33
tttcaggttc cttcgcatcg gactctggtt cctaag                                36

<210>  34

<211>  36

<212>  DNA

<213>  Artificial Sequence

<220>

<223>  fMptD5; generated by phage panning; frequency of occurrence: 1

<400>  34
acttcgcctc cgcctaatcg gaatcctagt gcgact                                36

<210>  35

<211>  36

<212>  DNA

<213>  Artificial Sequence

<220>

<223>  fMptD6; generated by phage panning; frequency of occurrence: 1

<400>  35
actctgacgt ctcagcatag tgctttggcg tatttg                                36

<210>  36

<211>  36

<212>  DNA

<213>  Artificial Sequence

<220>

<223>  fMptD7; generated by phage panning; frequency of occurrence: 1

<400>  36
gagcatatgg cgcttacgta tccgtttcgg ccgcct                                36

<210>  37

<211>  36

<212>  DNA

<213>  Artificial Sequence

<220>

<223>  fMptD8; generated by phage panning; frequency of occurrence: 1

<400>  37
attactcctt ctcattggac tcggttgctg acgcgg                                36

<210>  38

<211>  36

<212>  DNA

<213>  Artificial Sequence



<220>

<223>  fMptD9; enerated by phage panning; frequency of occurrence: 1

<400>  38
gataagccgt cgcctaggga tcgtgattcg tatccg                                36


<210>  39

<211>  36

<212>  DNA

<213>  Artificial Sequence



<220>

<223>  fMptD10; generated by phage panning; frequency of occurrence: 1

<400>  39
aagccgttga atactcctgt gttggtgcct acgaag                                36


<210>  40

<211>  36

<212>  DNA

<213>  Artificial Sequence



<220>

<223>  fMptD11; generated by phage panning; frequency of occurrence: 1

<400>  40
aatggtgttc atactaggtc ttcttatatg atttct                                36


<210>  41

<211>  36

<212>  DNA

<213>  Artificial Sequence

<220>

<223>  fMptD12; generated by phage panning; frequency of occurrence: 1

<400>  41
catttgccga agtataagct gcctccgggg atgggg                                36

<210>  42

<211>  36

<212>  DNA

<213>  Artificial Sequence

<220>

<223>  fMptD13; generated by phage panning; frequency of occurrence: 1

<400>  42
catacgtggc atcctcatat tcctccgcat tctagg                                36

<210>  43

<211>  36

<212>  DNA

<213>  Artificial Sequence

<220>

<223>  fMptD14; generated by phage panning; frequency of occurrence: 1

<400>  43
aatctgagta ttcttgggga gcatcagagg agtact                                36

<210>  44

<211>  36

<212>  DNA

<213>  Artificial Sequence

<220>

<223>  fMptD15; generated by phage panning; frequency of occurrence: 1

<400>  44
atgcttgata aggctcagtc tgtgtcggcg catcct                                36

<210> 45

<211> 36

<212> DNA

<213> Artificial Sequence

<220>

<223> fMptD16; generated by phage panning; frequency of occurrence: 1

<400> 45
atgccgattg atacttcgtt tcataagact gatcat 36

<210> 46

<211> 36

<212> DNA

<213> Artificial Sequence

<220>

<223> fMptD17; generated by phage panning; frequency of occurrence: 1

<400> 46
gttcatcctc tggatcggga tcgtcagcgg ggtccg 36

<210> 47

<211> 36

<212> DNA

<213> Artificial Sequence

<220>

<223> fMptD18; generated by phage panning; frequency of occurrence: 1

<400> 47
catacgtctt attcgcatgc tcgtcagcct actgtt 36

<210> 48

<211> 36

<212> DNA

<213> Artificial Sequence

<220>

<223>  fMptD19; generated by phage panning; frequency of occurrence: 1

<400>  48
aagaatcata tgagtcctcc tcatcgtggt ccttat                                36


<210>  49

<211>  36

<212>  DNA

<213>  Artificial Sequence



<220>

<223>  fMptD20; generated by phage panning; frequency of occurrence: 1

<400>  49
tctgggcagc ctccgcagtc tcggtctttt ttgttt                                36


<210>  50

<211>  36

<212>  DNA

<213>  Artificial Sequence



<220>

<223>  fMptD21; generated by phage panning; frequency of occurrence: 1

<400>  50
actcctccgg ctttggcgtc gccttttgcg cgttat                                36


<210>  51

<211>  36

<212>  DNA

<213>  Artificial Sequence



<220>

<223>  fMptD22; generated by phage panning; frequency of occurrence: 1

<400>  51
actagttata atatttttcc taggcatccg tttctt                                36

<210>  52

<211>  36

<212>  DNA

<213>  Artificial Sequence

<220>

<223>  fMptD23; generated by phage panning; frequency of occurrence: 1

<400>  52
aagcttaagt ctatgttgac gggtcgttct aatctt                                36

<210>  53

<211>  36

<212>  DNA

<213>  Artificial Sequence

<220>

<223>  fMptD24; generated by phage panning; frequency of occurrence: 1

<400>  53
gatcttaagt ctatgttgac gggtcgttct aatctt                                36

<210>  54

<211>  36

<212>  DNA

<213>  Artificial Sequence

<220>

<223>  fMptD25; generated by phage panning; frequency of occurrence: 1

<400>  54
cattatccgc cgtggacgca tgcttttagg gctggg                                36

<210>  55

<211>  36

<212>  DNA

<213>  Artificial Sequence

<220>

<223>  fMptD26; generated by phage panning; frequency of occurrence: 1

<400>  55
gggtataagt tgtcgaatag tgagtttaat ccgcct                                36

<210>  56

<211>  36

<212>  DNA

<213>  Artificial Sequence

<220>

<223>  fMptD27; generated by phage panning; frequency of occurrence: 1

<400>  56
atgaagactc cggggctgag tcagaatgtt gctcat                                36

<210>  57

<211>  36

<212>  DNA

<213>  Artificial Sequence

<220>

<223>  fMptD28; generated by phage panning; frequency of occurrence: 1

<400>  57
actcgggttc atgttattga tctgccgcgg gtgtgg                                36

<210>  58

<211>  36

<212>  DNA

<213>  Artificial Sequence

<220>

<223>  fMptD29; generated by phage panning; frequency of occurrence: 1

<400>  58
gatagggcta ctccgtgggg gaatttgccg aggtct                                36

<210> 59

<211> 36

<212> DNA

<213> Artificial Sequence

<220>

<223> fMptD30; generated by phage panning; frequency of occurrence: 1

<400> 59
catctgaatc atgatatgaa gcggtggccg actgcg 36

<210> 60

<211> 36

<212> DNA

<213> Artificial Sequence

<220>

<223> fMptD31; generated by phage panning; frequency of occurrence: 1

<400> 60
catgagtata ctgctaaggt tcctcgtctg atttct 36

<210> 61

<211> 36

<212> DNA

<213> Artificial Sequence

<220>

<223> fMptD32; generated by phage panning; frequency of occurrence: 1

<400> 61
ggtactatta agggtgcgtc gactacgcgg ttgccg 36

<210> 62

<211> 36

<212> DNA

<213> Artificial Sequence

<220>

<223>  fMptD33; generated by phage panning; frequency of occurrence: 1

<400>  62
ttgcctcatg agcatgcgcc gacgaggcct ccgcgg                                36


<210>  63

<211>  12

<212>  PRT

<213>  Artificial Sequence



<220>

<223>  corresponds to fMptD1

<400>  63

Gly Lys Asn His His His Gln His His Arg Pro Gln
1               5                   10


<210>  64

<211>  12

<212>  PRT

<213>  Artificial Sequence



<220>

<223>  corresponds to fMptD2

<400>  64

Trp Pro Ser Trp Thr Ser Thr Ser Gly Leu Thr Lys
1               5                   10


<210>  65

<211>  12

<212>  PRT

<213>  Artificial Sequence



<220>

<223>  corresponds to fMptD3

<400> 65

Glu Ile Leu Trp Ser Arg Val Pro Ala Thr Thr Arg
1               5                   10

<210> 66

<211> 12

<212> PRT

<213> Artificial Sequence

<220>

<223> corresponds to fMptD4

<400> 66

Phe Gln Val Pro Ser His Arg Thr Leu Val Pro Lys
1               5                   10

<210> 67

<211> 12

<212> PRT

<213> Artificial Sequence

<220>

<223> corresponds to fMptD5

<400> 67

Thr Ser Pro Pro Pro Asn Arg Asn Pro Ser Ala Thr
1               5                   10

<210> 68

<211> 12

<212> PRT

<213> Artificial Sequence

<220>

<223> corresponds to fMptD6

<400> 68

Thr Leu Thr Ser Gln His Ser Ala Leu Ala Tyr Leu
1               5                   10

<210>  69

<211>  12

<212>  PRT

<213>  Artificial Sequence

<220>

<223>  corresponds to fMptD7

<400>  69

Glu His Met Ala Leu Thr Tyr Pro Phe Arg Pro Pro
1               5                   10

<210>  70

<211>  12

<212>  PRT

<213>  Artificial Sequence

<220>

<223>  corresponds to fMptD8

<400>  70

Ile Thr Pro Ser His Trp Thr Arg Leu Leu Thr Arg
1               5                   10

<210>  71

<211>  12

<212>  PRT

<213>  Artificial Sequence

<220>

<223>  corresponds to fMptD9

<400>  71

Asp Lys Pro Ser Pro Arg Asp Arg Asp Ser Tyr Pro
1               5                   10

<210> 72

<211> 12

<212> PRT

<213> Artificial Sequence

<220>

<223> corresponds to fMptD10

<400> 72

Lys Pro Leu Asn Thr Pro Val Leu Val Pro Thr Lys
1               5                   10

<210> 73

<211> 12

<212> PRT

<213> Artificial Sequence

<220>

<223> corresponds to fMptD11

<400> 73

Asn Gly Val His Thr Arg Ser Ser Tyr Met Ile Ser
1               5                   10

<210> 74

<211> 12

<212> PRT

<213> Artificial Sequence

<220>

<223> corresponds to fMptD12

<400> 74

His Leu Pro Lys Tyr Lys Leu Pro Pro Gly Met Gly
1               5                   10

<210> 75

<211>  12

<212>  PRT

<213>  Artificial Sequence

<220>

<223>  corresponds to fMptD13

<400>  75

His Thr Trp His Pro His Ile Pro Pro His Ser Arg
1               5                   10

<210>  76

<211>  12

<212>  PRT

<213>  Artificial Sequence

<220>

<223>  corresponds to fMptD14

<400>  76

Asn Leu Ser Ile Leu Gly Glu His Gln Arg Ser Thr
1               5                   10

<210>  77

<211>  12

<212>  PRT

<213>  Artificial Sequence

<220>

<223>  corresponds to fMptD15

<400>  77

Met Leu Asp Lys Ala Gln Ser Val Ser Ala His Pro
1               5                   10

<210>  78

<211>  12

<212>  PRT

<213>  Artificial Sequence



<220>

<223>  corresponds to fMptD16

<400>  78

Met Pro Ile Asp Thr Ser Phe His Lys Thr Asp His
1               5                   10


<210>  79

<211>  12

<212>  PRT

<213>  Artificial Sequence



<220>

<223>  corresponds to fMptD17

<400>  79

Val His Pro Leu Asp Arg Asp Arg Gln Arg Gly Pro
1               5                   10


<210>  80

<211>  12

<212>  PRT

<213>  Artificial Sequence



<220>

<223>  corresponds to fMptD18

<400>  80

His Thr Ser Tyr Ser His Ala Arg Gln Pro Thr Val
1               5                   10


<210>  81

<211>  12

<212>  PRT

<213>  Artificial Sequence

<220>

<223>  corresponds to fMptD19

<400>  81

Lys Asn His Met Ser Pro Pro His Arg Gly Pro Tyr
1               5                   10

<210>  82

<211>  12

<212>  PRT

<213>  Artificial Sequence

<220>

<223>  corresponds to fMptD20

<400>  82

Ser Gly Gln Pro Pro Gln Ser Arg Ser Phe Leu Phe
1               5                   10

<210>  83

<211>  12

<212>  PRT

<213>  Artificial Sequence

<220>

<223>  corresponds to fMptD21

<400>  83

Thr Pro Pro Ala Leu Ala Ser Pro Phe Ala Arg Tyr
1               5                   10

<210>  84

<211>  12

<212>  PRT

<213>  Artificial Sequence

<220>

<223>  corresponds to fMptD22

<400>  84

Thr Ser Tyr Asn Ile Phe Pro Arg His Pro Phe Leu
1               5                   10


<210>  85

<211>  12

<212>  PRT

<213>  Artificial Sequence



<220>

<223>  corresponds to fMptD23

<400>  85

Lys Leu Lys Ser Met Leu Thr Gly Arg Ser Asn Leu
1               5                   10


<210>  86

<211>  12

<212>  PRT

<213>  Artificial Sequence



<220>

<223>  corresponds to fMptD24

<400>  86

Asp Leu Lys Ser Met Leu Thr Gly Arg Ser Asn Leu
1               5                   10


<210>  87

<211>  12

<212>  PRT

<213>  Artificial Sequence

<220>

<223>  corresponds to fMptD25

<400>  87

His Tyr Pro Pro Trp Thr His Ala Phe Arg Ala Gly
1               5                   10


<210>  88

<211>  12

<212>  PRT

<213>  Artificial Sequence



<220>

<223>  corresponds to fMptD26

<400>  88

Gly Tyr Lys Leu Ser Asn Ser Glu Phe Asn Pro Pro
1               5                   10


<210>  89

<211>  12

<212>  PRT

<213>  Artificial Sequence



<220>

<223>  corresponds to fMptD27

<400>  89

Met Lys Thr Pro Gly Leu Ser Gln Asn Val Ala His
1               5                   10


<210>  90

<211>  12

<212>  PRT

<213>  Artificial Sequence



<220>

<223>  corresponds to fMptD28

<400>  90

Thr Arg Val His Val Ile Asp Leu Pro Arg Val Trp
1               5                   10


<210>  91

<211>  12

<212>  PRT

<213>  Artificial Sequence



<220>

<223>  corresponds to fMptD29

<400>  91

Asp Arg Ala Thr Pro Trp Gly Asn Leu Pro Arg Ser
1               5                   10


<210>  92

<211>  12

<212>  PRT

<213>  Artificial Sequence



<220>

<223>  corresponds to fMptD30

<400>  92

His Leu Asn His Asp Met Lys Arg Trp Pro Thr Ala
1               5                   10


<210>  93

<211>  12

<212>  PRT

<213>  Artificial Sequence



<220>

<223>  corresponds to fMptD31

<400>  93

His Glu Tyr Thr Ala Lys Val Pro Arg Leu Ile Ser
1               5                   10

<210>  94

<211>  12

<212>  PRT

<213>  Artificial Sequence

<220>

<223>  corresponds to fMptD32

<400>  94

Gly Thr Ile Lys Gly Ala Ser Thr Thr Arg Leu Pro
1               5                   10

<210>  95

<211>  12

<212>  PRT

<213>  Artificial Sequence

<220>

<223>  corresponds to fMptD33

<400>  95

Leu Pro His Glu His Ala Pro Thr Arg Pro Pro Arg
1               5                   10

## Beschreibung

Die vorliegende Erfindung betrifft Mittel und Verfahren zum Anreichern und Nachweisen von Mikroorganismen. Die Erfindung betrifft insbesondere Anreicherungsverfahren und Immobilisierungsverfahren zum Immobilisieren von Mikroorganismen, sowie dazu vorteilhafte Peptide, Reagenzien und Kits. Die Erfindung betrifft ferner Nachweisverfahren zum Nachweisen von Mikroorganismen, sowie dazu vorteilhafte Reagenzien und Kits. Ferner betrifft die Erfindung Verfahren zum Herstellen von Peptiden, die an Mikroorganismen binden können, sowie dazu besonders geeignete Puffer und Nucleinsäuren. Die Erfindung betrifft insbesondere auch Anreicherungs- und Nachweisverfahren zum Anreichern und Nachweisen von *Mycobacterium paratuberculosis* sowie in diesem Zusammenhang vorteilhafte Peptide.

In der Mikrobiologie und in der klinischen Diagnostik stellt sich häufig das Problem, einen Mikroorganismus nachzuweisen. Unter einem Mikroorganismus wird hier ein mit bloßem Auge gewöhnlich nicht sichtbares Kleinlebewesen verstanden. Viele Mikroorganismen können beispielsweise bei einer Hinterlegungsstelle nach dem Budapester Vertrag über die internationale Anerkennung der Hinterlegung von Mikroorganismen für die Zwecke von Patentverfahren hinterlegt werden. Zu den Mikroorganismen gehören insbesondere Viren, Eubacterien, Archaebacterien, Pilze, Protozooen und sonstige eukaryontische Zellen. Einige Mikroorganismen, beispielsweise Mycobacterien, sind für den Menschen insbesondere deshalb von Bedeutung, weil sie beim Menschen oder Nutztieren Krankheiten auslösen können oder krankheitsbegleitend auftreten.

Zum Nachweis von Mikroorganismen werden zahlreiche unterschiedliche Verfahren verwendet. Mikroskopie-Verfahren beruhen im wesentlichen auf dem optischen oder elektronenmikroskopischen Nachweis der Mikroorganismen, beispielsweise unter Verwendung besonderer Färbeverfahren wie Gram-Färbung, Ziehl-Neelsen-Färbung und ähnlichen. Kultivierungsverfahren machen sich die Tatsache zunutze, dass Mikroorganismen bestimmte Nährsubstrate für ihr Wachstum benötigen, bzw. dass bestimmte Substanzen das Wachstum hemmen können. Für zahlreiche Mikroorganismen sind Kultivierungs- und Anreicherungsverfahren unter Verwendung spezieller Nährsubstrate und Kultivierungsbedingungen (Temperatur, Zusammensetzung der Atmosphäre und dergleichen) beschrieben; durch den Vergleich des Wachstumsverhaltens auf verschiedenen Nährsubstraten bzw. unter verschiedenen Kultivierungsbedingungen ist es häufig auch möglich, Mikroorganismen der Gattung oder Art nach zu identifizieren. Mit molekularbiologischen und immunochemischen Verfahren wird versucht, bestimmte Bestandteile von Mikroorganismen, wie Nucleinsäuren und Antigene, nachzuweisen; in diesem Zusammenhang sind insbesondere PCR- und Hybridisierungsverfahren, ELISA, fluoreszenz-aktivierte Zellsortierung (FACS) und Blotting-Verfahren wie Western Blots bekannt.

Häufig ist jedoch problematisch, dass die nachzuweisenden Mikroorganismen in einer zu untersuchenden Probe nur in geringer Konzentration vorliegen. Auch wirken in der Probe vorhandene Begleitsubstanzen häufig störend auf das eingesetzte Nachweisverfahren. Beispielsweise besitzen Milch-, Kot-, Boden- und Sedimentproben eine komplexe Zusammensetzung aus zahlreichen makromolekularen Substanzen, die die Durchführung einiger Nachweisverfahren wie FACS unmöglich machen oder die Sensitivität und/oder Spezifität von Nachweisverfahren wie beispielsweise PCR-basierter Verfahren verschlechtern. Proben enthalten häufig auch nicht nur Mikroorganismen der nachzuweisenden Art, sondern auch andere Mikroorganismen; diese wiederum können, beispielsweise durch Kreuzreaktionen, die Sensitivität und/oder Spezifität von Nachweisverfahren ebenfalls verschlechtern. Manche Mikroorganismen haben eine sehr lange Generationszeit, so dass ein rascher Nachweis durch Kultivierungsverfahren oder der Analyse ihrer metabolischen Aktivität nur unter großen Schwierigkeiten möglich ist. Immunochemische Nachweisverfahren wiederum haben den Nachteil, dass häufig keine sensitiven und/oder spezifischen Antikörper für den nachzuweisenden Mikroorganismus zur Verfügung stehen, oder dass solche Antikörper nur schwer herzustellen und/oder teuer sind. Unter Antikörpern werden hier auch deren verwandte Immunglobuline sowie Teilpeptide (Fragmente) wie Fab, F(ab)₂ und single-chain Antikörper verstanden; eine Übersicht über Immunglobuline und deren Teilpeptide ist beispielsweise in Alberts et al., Molecular Biology of the Cell, 3. Auflage 1994, zu finden.

Es war eine Aufgabe der Erfindung, die beschriebenen und gegebenenfalls auch weitere Nachteile zu mindern oder diese ganz auszuschalten. Die Erfindung sollte insbesondere einfache und möglichst schnell durchführbare Verfahren zum Anreichern, Immobilisieren und/oder Nachweisen von Mikroorganismen sowie dazu vorteilhafte Reagenzien und Reagenzien-Zusammenstellungen (Kits) angeben. Ferner war es eine Aufgabe der Erfindung, Verfahren und Reagenzien anzugeben, mit denen auf möglichst einfache Weise weitere Reagenzien zur Verwendung in einem dieser Anreicherungs-, Immobilisierungs- und/oder Nachweisverfahren hergestellt werden können.

Gemäß einem Aspekt der Erfindung wird daher ein Fängerpeptid angegeben, das dadurch gekennzeichnet ist, dass
a) das Fängerpeptid an einen Mikroorganismus binden kann, und dass
b) das Fängerpeptid aus 5 bis 20 Aminosäuren besteht.

Häufig werden Mikroorganismen in immunochemischen Verfahren unter Verwendung von Antikörpern nachgewiesen und/oder immobilisiert; beispielsweise in Sandwich-ELISA-Verfahren. Antikörper lassen sich jedoch nicht oder nur unter großen Schwierigkeiten für jeden nachzuweisenden oder zu immobilisierenden Mikroorganismus herstellen. Sie setzen zudem praktisch immer die Exposition von Versuchstieren mit dem Mikroorganismus oder Teilen desselben voraus, wobei aus dem Versuchstier Antikörperproduzierende Zellen gewonnen werden. Dieses Verfahren ist nur schwer reproduzierbar, die Anreicherung und Immortalisierung der Antikörper-produzierenden Zellen ist aufwendig und gelingt nicht immer, und die Gewinnung der Antikörper aus den Antikörper-produzierenden Zellen ist häufig ebenfalls schwierig, so dass nur geringe Ausbeuten an Antikörpern zu einem hohen Preis erzielt werden können. Häufig tritt auch das Problem auf, dass nicht ein einzelner, spezifischer Antikörper (monoklonaler Antikörper) erhalten wird, sondern nur eine Mischung verschiedener Antikörper (polyklonaler Antikörper). Die Stabilität, Sensitivität und Spezifität der einzelnen Antikörper einer solchen Mischung kann durchaus unterschiedlich sein und die Auswertung von Nachweisverfahren erschweren. Zudem sind Antikörper empfindlich gegenüber Änderungen der Zusammensetzung von Lösungen und Puffern. Sie büßen ihre Aktivität oftmals schon beim Einfrieren und Auftauen ein, so dass ihre Stabilität, Lagerungsfähigkeit und Handhabbarkeit eingeschränkt ist.

Die Erfindung setzt an der Erkenntnis ein, dass Mikroorganismen gewöhnlich eine komplexe Oberfläche, beispielsweise eine Zellmembran, Zellhülle oder Kapside besitzen. Es hat sich nunmehr herausgestellt, dass bereits kurze Peptide von 5 bis 20 Aminosäuren Länge fest und mit hoher Spezifität an Bestandteile der komplexen Oberfläche binden können, so dass die Herstellung und Verwendung von Antikörpern in vielen Fällen auf vorteilhafte Weise vermieden werden kann. Solche kurzen, an Mikroorganismen bindenden Peptide (Fängerpeptide) lassen sich zudem mit herkömmlichen Syntheseverfahren leicht herstellen. Daher kann eine gleichbleibende, chargenunabhängige Produktqualität erzielt werden, insbesondere im Hinblick auf die Reinheit und Stabilität. Die Handhabung der in Immobilisierungsund/oder Nachweisverfahren einzusetzenden Reagenzien wird ebenfalls vereinfacht, so dass insgesamt die Immobilisierung und/oder der Nachweis von Mikroorganismen schneller, mit höherer Reproduzierbarkeit und mit geringerem Gesamtaufwand ermöglicht wird. Zudem ist es einfacher, Nachweis- und/oder lmmobilisierungsverfahren zu miniaturisieren und zu automatisieren, in denen statt Antikörpern Fängerpeptide verwendet werden.

Darüber hinaus ermöglichen es Fängerpeptide, auch kurzfristige Änderungen im physiologischen Zustand von Mikroorganismen nachzuweisen. Beispielsweise durchlaufen Mikroorganismen bei ihrer Vermehrung unterschiedliche Stadien, die sich in unterschiedlicher Expression und Expressionsstärke ihrer Gene und damit in kurz währenden Änderungen der Konzentration und/oder Konformation der entsprechenden Genprodukte niederschlägt. Die Gewinnung von Antikörpern gegen solche Genprodukte bzw. deren Konformationen ohne eine vorherige genaue Kenntnis der betreffenden Genprodukte und gegebenenfalls deren spezielle Konformationen ist wegen des flüchtigen Charakters der nachzuweisenden Veränderungen praktisch nicht möglich. Kurze synthetische Peptide können jedoch in einer hohen Sequenzvielfalt auf Vorrat in Form einer Peptid-Bibliothek, beispielsweise einer Phagen-Display-Bank oder eines Peptid-Microarrays, hergestellt werden. Aus der Peptid-Bibliothek können in einem an den jeweiligen Mikroorganismus und die jeweilige Peptid-Bibliothek angepassten Screening-Verfahren solche Fängerpeptide selektiert werden, die an den gesuchten Mikroorganismus, gegebenenfalls im interessierenden physiologischen Zustand, binden können.

Vorzugsweise besteht das Fängerpeptid aus 8 bis 14 Aminosäuren. Es hat sich herausgestellt, dass bereits derartig kurze Fängerpeptide ein sicheres und spezifisches Binden an nachzuweisende Mikroorganismen ermöglichen können. Zudem sind solche Peptide leicht handhabbar und unter nur geringen Kosten zu synthetisieren.

Erfindungsgemäß wird ferner ein Fängerreagens angegeben, umfassend
a) ein Fängerpeptid der zuvor beschriebenen Art, gebunden an
b) einen festen Träger.

Indem das Fängerpeptid an einen festen Träger gebunden ist, wird es auf vorteilhaft einfache Weise möglich, Mikroorganismen aus Flüssigkeiten zu gewinnen, anzureichern und zu immobilisieren.

Als fester Träger können insbesondere die in herkömmlichen Anreicherungs- bzw. Nachweisverfahren verwendeten festen Träger eingesetzt werden. Feste Träger können insbesondere Metallkörper sein, beispielsweise Goldfolien und goldbeschichtete Träger wie im Biacore-System, oder paramagnetische Träger wie paramagnetische Beads. Ferner können Kunststoffkörper bzw. kunststoffbeschichtete Körper als feste Träger verwendet werden, beispielsweise Polystyrol- oder Polymethylmethacrylat-Träger, wie auch Mikrotiterplatten. Daneben können auch Glasträger, Gele oder andere, zur Herstellung von Biochips geeignete Träger, verwendet werden. Die genannten Träger werden in zahlreichen Labortechniken verwendet, so dass ihre Handhabung bereits eingeübt ist.

Das Fängerpeptid kann durch verschiedene, dem Fachmann bekannte Verfahren an den festen Träger gebunden werden. Beispielsweise kann das Fängerpeptid durch Adsorption an den festen Träger gebunden werden. Der feste Träger kann auch mit einer Beschichtung versehen sein, die die Bindung des Fängerpeptids bewirkt. Beispielsweise kann der Träger Streptavidinbeschichtet sein, so dass ein biotinyliertes Fängerpeptid fest gebunden werden kann. Andere mögliche Beschichtungen zum Binden eines Fängerpeptids sind solche, die Serumalbumin, Protein A und/oder Protein G umfassen. Das Peptid kann auch mit einer Nucleinsäure verbunden sein, die über eine an dem festen Träger gebundene komplementäre Nucleinsäure die Bindung des Peptids an den Träger vermittelt.

Besonders vorteilhaft ist es, wenn der feste Träger des Fängerreagens partikelförmig ist. Partikelförmige Träger lassen sich leicht mit daran immobilisierten Mikroorganismen aus Lösungen und Aerosolen abtrennen; dies kann beispielsweise bei den besonders bevorzugten paramagnetischen Partikeln (Beads) durch Anlegen eines äußeren Magnetfeldes geschehen. Für viele Partikel reicht zur Abtrennung bereits eine einfache Sedimentation oder schonende Zentrifugation aus. Zudem besitzen partikelförmige Träger eine hohe verfügbare Oberfläche, so dass ein hoher Anteil des Probenvolumens in Kontakt mit dem Träger tritt. Der Umgang mit partikelförmigen Trägern wie beispielsweise paramagnetischen Beads und Verfahren zum Binden von Peptiden an partikelförmige Träger sind bereits in zahlreichen Labors bekannt. Die verwendeten Materialien sind preiswert und die Handhabung ist einfach.

Gemäß einem weiteren Aspekt der Erfindung wird ein lmmobilisierungsverfahren zum Immobilisieren eines Mikroorganismus angegeben, umfassend die Schritte:
a) Binden eines Fängerpeptids der zuvor beschriebenen Art an einen festen Träger, und
b) Inkontaktbringen des Fängerpeptids mit einer zu untersuchenden Probe, um ein Binden des Fängerpeptids an den zu immobilisierenden Mikroorganismus zu ermöglichen.

Das Fängerpeptid bindet sowohl an den festen Träger als auch an den zu immobilisierenden Mikroorganismus. Dadurch stellt es auf vorteilhaft einfache Weise eine stabile und spezifische Verbindung zwischen dem festen Träger und dem zu immobilisierenden Mikroorganismus her. Der Mikroorganismus kann anschließend einfach von der ihn enthaltenden Probe abgetrennt werden, beispielsweise indem der Träger absedimentiert, abzentrifugiert oder durch Anlegen eines äußeren Magnetfeldes abgetrennt wird. Wenn der feste Träger ein Gefäß zur Aufnahme der Probe selbst ist, beispielsweise in Form einer Mikrotiterplatten-Vertiefung, so können die gebundenen Mikroorganismen durch Waschen des Gefäßes mit einer entsprechenden Waschlösung von der übrigen Probe abgetrennt und somit aufgereinigt und angereichert werden. Zweckmäßigerweise ist der feste Träger partikelförmig, so dass die vorstehend beschriebenen, mit der Verwendung partikelförmiger fester Träger verbundenen Vorteile ausgenutzt werden können.

Dabei ist die Reihenfolge, in der die Schritte a) und b) durchgeführt werden, nicht festgelegt. Es hat sich gezeigt, dass die Bindung von Fängerpeptiden an einen zu immobilisierenden Mikroorganismus behindert sein kann, wenn das Fängerpeptid vor der Bindung an den Mikroorganismus bereits an den festen Träger gebunden ist. In einem solchen Fall ist es vorteilhaft, den Schritt b) vor oder gleichzeitig mit dem Schritt a) durchzuführen, indem beispielsweise das Fängerpeptid und die zu untersuchende Probe durch Mischen miteinander in Kontakt gebracht werden, und gleichzeitig oder anschließend der feste Träger mit der Mischung in Kontakt gebracht wird. Wenn jedoch der Schritt a) vor dem Schritt b) durchgeführt wird, beispielsweise indem ein erfindungsgemäßes Fängerreagens wie zuvor beschrieben verwendet wird, so kann derselbe Träger mehrfach zum Immobilisieren von Mikroorganismen aus mehreren Proben oder zum Immobilisieren von Mikroorganismen aus einem Probenstrom verwendet werden. Ein solches Vorgehen kann insbesondere dann vorteilhaft sein, wenn die zu untersuchenden Proben nur geringe Konzentrationen des nachzuweisenden Mikroorganismus besitzen, oder wenn die Proben zahlreiche Begleitsubstanzen enthalten, die unspezifisch an den festen Träger binden oder auf andere Weise die Bindung der Fängerpeptide an den nachzuweisenden Mikroorganismus stören.

Ferner werden erfindungsgemäß Bindungspeptide angegeben, die
a) einen 8 Aminosäuren langen Ausschnitt aus einer Aminosäuresequenz gemäß einer der Sequenzen SEQ ID NO. 1 bis SEQ ID NO. 9 oder SEQ ID NO. 63 bis SEQ ID NO. 95 oder
b) einen zu einem Ausschnitt gemäß a) funktionsgleichen, 8 Aminosäuren langen Ausschnitt umfassen.

Es hat sich überraschenderweise herausgestellt, dass diese Bindungspeptide besonders gut geeignet sind, an *Mycobacterium paratuberculosis (M. paratuberculosis)* zu binden.

*Mycobacterium paratuberculosis* wird in die Ordnung der *Actinomycetales* eingeordnet und als Subspezies von *Mycobacterium avium* klassifiziert. *M. paratuberculosis* ist ein säurefestes, unbewegliches, schwach grampositives Stäbchen. Es verursacht Paratuberkulose bei Rindern und wird in Zusammenhang mit Morbus Crohn beim Menschen gebracht. Die Paratuberkulose hat eine lange Inkubationszeit und verläuft meist subklinisch, wobei der Erreger intermittierend mit dem Kot und der Milch ausgeschieden wird. Die subklinisch infizierten Tiere können den Erreger über Jahre hinweg ausscheiden. *M. paratuberculosis* ist ein sehr widerstandsfähiger Keim, der die kommerzielle Milch-Pasteurisierung überlebt.

Die Diagnose subklinisch erkrankter Rinder ist schwierig. Mikroskopische Verfahren wie die Ziehl-Neelsen-Färbung haben nur eine geringe Bedeutung, da sie eine Erregerkonzentration von mehr als 10⁴ Keimen pro Gramm Kot für den Nachweis erfordern.

Traditionell erfolgt der Nachweis von *M. paratuberculosis* vor allem durch Kultivierung auf eidotterhaltigen Nährböden. Mit solchen Verfahren kann zwar eine hohe Sensitivität erreicht werden, allerdings benötigt *M. paratuberculosis* eine Kultivierungsdauer von 2 Monaten und länger. Für einen raschen Nachweis einer Kontamination in einer Probe, beispielsweise in Milch, sind Kultivierungsverfahren daher nicht geeignet.

Zuverlässige immunologische Nachweisverfahren sind bisher vor allem daran gescheitert, dass es nicht gelungen ist, monoklonale Antikörper gegen das Hauptantigen von *M. paratuberculosis,* das Lipoarabinomannan (LAM), zu gewinnen.

Nachweisverfahren unter Verwendung von PCR haben sich bisher als nicht sensitiv genug erwiesen, die Nachweisgrenzen liegen zwischen 30 und 300 Erregern pro Milliliter Milch.

Es besteht daher ein Bedarf nach Mitteln, um *M. paratuberculosis* auf möglichst einfache, sensitive und spezifische Weise auch in komplexen Proben wie Kot und Milch nachweisen zu können. Die erfindungsgemäß angegebenen Bindungspeptide sind überraschenderweise geeignet, an *M. paratuberculosis* zu binden und somit in einem Anreicherungs- bzw. Nachweisverfahren zur Anreicherung und/oder zum Nachweisen von *M. paratuberculosis* eingesetzt zu werden.

Die Bindungspeptide umfassen einen zumindest 8 Aminosäuren langen Aussschnitt aus einer der Aminosäuresequenzen gemäß SEQ ID NO 1 bis SEQ ID NO 9 oder SEQ ID NO. 63 bis SEQ ID NO. 95. Dabei versteht es sich, dass die Bindungspeptide auch länger als 8 Aminosäuren sein können. Insbesondere können sie die volle Länge der in SEQ ID NO 1 bis SEQ ID NO 9 bzw. SEQ ID NO. 63 bis SEQ ID NO. 95 angegebenen Peptide umfassen.

Die Bindungspeptide sind auch nicht darauf festgelegt, exakt einen zumindest 8 Aminosäuren langen Ausschnitt einer der Sequenzen SEQ ID NO 1 bis SEQ ID NO 9 oder SEQ ID NO. 63 bis SEQ ID NO. 95 zu umfassen; stattdessen können sie auch einen Abschnitt umfassen, der einem solchen Ausschnitt funktionsgleich ist, also ebenfalls spezifisch an *M. paratuberculosis* binden kann. Beispielsweise können in manchen Bindungspeptiden die Aminosäuren Isoleucin und Leucin gegeneinander ausgetauscht werden. Andere Änderungen sind ebenfalls möglich, ohne die Funktion der Bindungspeptide aufzuheben.

In bevorzugten Ausgestaltungen sind die Bindungspeptide Fängerpeptide, also maximal 20 Aminosäuren lang. Solche kurzen Bindungspeptide verwirklichen die eingangs für Fängerpeptide beschriebenen Vorteile.

Bevorzugt sind solche Bindungspeptide, die einen zumindest 8 Aminosäuren langen Ausschnitt einer Aminosäuresequenz gemäß SEQ ID NO 3, SEQ ID NO 8 und/oder SEQ ID NO. 63 oder einen funktionsgleichen Ausschnitt umfassen. Diese Peptide haben sich in ersten Versuchen als besonders geeignet zum Binden an *M. paratuberculosis* herausgestellt. Insbesondere besitzen sie eine hohe Affinität für *M. paratuberculosis.* Sie können auch in komplexen Proben spezifisch und mit hoher Ausbeute an *M. paratuberculosis* binden.

Besonders bevorzugt sind Bindungspeptide der Aninosäuresequenz SEQ ID NO 3, SEQ ID NO 8 oder SEQ ID NO 63. Diese Bindungspeptide sind gemäß ersten Versuchsergebnissen besonders spezifisch für *M. paratuberculosis* und weisen eine hohe Affinität für diesen Mikroorganismus auf. Sie sind in besonderer Weise geeignet, an *M. paratuberculosis* in Milch- und Kotproben zu binden. Sie verwirklichen zudem die übrigen Vorteile, die Bindungs- und Fängerpeptide bieten.

Erfindungsgemäß wird auch ein Anreicherungsverfahren zum Anreichern von *M. paratuberculosis* angegeben, umfassend die Schritte:
a) Binden eines Bindungspeptids wie zuvor beschrieben an einen festen Träger, und
b) Inkontaktbringen des Bindungspeptids mit einer zu untersuchenden Probe, um ein Binden des Bindungspeptids an *M. paratuberculosis* zu ermöglichen.

Ein solches Anreicherungsverfahren verwirklicht die oben beschriebenen Vorteile eines erfindungsgemäßen lmmobilisierungsverfahrens. Zudem erlaubt das Anreicherungsverfahren, auch aus stark verdünnten Proben genügend hohe Zellzahlen von *M. paratuberculosis* zu isolieren, um einen sicheren und spezifischen Nachweis des Erregers zu ermöglichen. Wiederum ist die Verwendung partikelförmiger fester Träger wegen der genannten Vorteile besonders bevorzugt.

Dabei hängt es wiederum von der Art der zu untersuchenden Probe ab, in welcher Reihenfolge die Schritte a) und b) durchgeführt werden. Der Fachmann kann die für die jeweilige Probe günstigste Reihenfolge anhand weniger Vorversuche leicht selbst bestimmen. Wird Schritt a) vor Schritt b) durchgeführt, dann entspricht der in Schritt a) entstehende Verbund einem Fängerreagens der oben beschriebenen Art und kann insbesondere die mit einem solchen Fängerreagens erzielbaren Vorteile verwirklichen.

Der Fachmann kann ebenfalls leicht selbst bestimmen, in welcher Weise das Bindungspeptid an den festen Träger gebunden werden soll. Vorteilhafterweise wird das Bindungspeptid ohne Vermittlung eines besonderen Linkers wie Biotin-Streptavidin an den festen Träger gebunden. Die unmittelbare Bindung des Bindungspeptids an den festen Träger ist sehr preiswert und einfach durchzuführen. Das Bindungspeptid kann natürlich auch, wie ein Fängerpeptid, über einen Linker mit dem festen Träger verbunden werden, beispielsweise indem ein biotinyliertes Bindungspeptid an einen Streptavidinbeschichteten festen Träger gebunden wird.

Weiter wird ein Nachweisreagens zum Nachweisen eines Mikroorganismus angegeben, wobei das Nachweisreagens umfasst:
a) ein Fängerpeptid und/oder ein Bindungspeptid, jeweils nach einer der hierin beschriebenen Arten, verbunden mit
b) einem Marker.

Die Fänger- bzw. Bindungspeptide besitzen eine hohe Affinität für den nachzuweisenden Mikroorganismus. Sie lassen sich daher gut als Sonden für Mikroorganismen verwenden. Dazu werden sie vorteilhafterweise mit einem leicht nachweisbaren Marker versehen. Geeignete Marker sind dem Fachmann aus zahlreichen Anwendungen, beispielsweise aus ELISA, bekannt.

Beispielsweise kann der Marker partikelförmig sein. Die Bindung solcher Marker an einer Oberfläche ist optisch leicht nachzuweisen.

Als Marker können auch radioaktive, fluoreszierende, phosphoreszierende und/oder farbige Einheiten verwendet werden. Es gibt bereits gut etablierte Verfahren, solche Marker mit Peptiden zu verbinden und sie gegebenenfalls auch in komplexen Proben nachzuweisen. Besonders geeignet sind hierfür radioaktive und fluoreszierende Marker.

Vorzugsweise umfasst der Marker eine Nucleinsäure. Nucleinsäuren können leicht mit Fänger- bzw. Bindungspeptiden verbunden werden. Zudem lassen sie sich mit etablierten Verfahren, beispielsweise der Polymerase-Kettenreaktion (PCR), leicht vervielfältigen, so dass eine hohe Nachweisempfindlichkeit mit einem robusten Nachweisverfahren erreicht wird.

Der Marker kann ebenfalls eine katalytisch wirkende Einheit umfassen. Insbesondere ist es möglich, ein Fänger- bzw. Bindungspeptid mit einem Enzym zu vesehen, das die Synthese ein fluoreszierenden, phosphoreszierenden und/oder farbigen Produkts katalysiert. Aus der Bildung des Produkts kann dann auf die Menge des nachzuweisenden Mikroorganismus in der Probe geschlossen werden. Die katalytisch wirkende Einheit muss noch nicht während des Bindens des Fänger- bzw. Bindungspeptids an dem Mikroorganismus mit dem Fänger- bzw. Bindungspeptid verbunden sein, es ist auch möglich, den Marker erst nach der Bindung an den Mikroorganismus am Fänger- bzw. Bindungspeptid anzubringen. Dies kann beispielsweise durch Vermittlung eines Linkers wie Biotin-Streptavidin geschehen, indem das Fänger- bzw. Bindungspeptid sowie die katalytisch wirkende Einheit biotinyliert sind. Nach der Bindung des Fänger- bzw. Bindungspeptids an den nachzuweisenden Mikroorganismus werden Streptavidin und die biotinylierte katalytisch wirkende Einheit zugegeben. Streptavidin vermittelt dann die Bindung der katalytisch wirkenden Einheit an das Fänger- bzw. Bindungspeptid.

Gemäß einem weiteren Aspekt der Erfindung wird ein Nachweisverfahren zum Nachweisen eines Mikroorganismus angegeben. Das Nachweisverfahren umfasst die Schritte:
a) Inkontaktbringen einer zu untersuchenden Probe mit einem Nachweisreagens der zuvor beschriebenen Art, um ein Binden des Fänger- und/oder Bindungspeptids an den nachzuweisenden Mikroorganismus zu ermöglichen, und
b) Nachweisen des Markers des Nachweisreagens.

Das Nachweisverfahren verwirklicht die mit der Verwendung der erfindungsgemäßen Fänger- bzw. Bindungspeptide verbundenen Vorteile. Insbesondere kann es einen spezifischen und sensitiven Nachweis des Mikroorganismus auch in komplexen Proben wie Milch und Kot ermöglichen. Aus den oben beschriebenen Gründen ist es zudem besonders bevorzugt, wenn der Marker des Nachweisreagens eine Nucleinsäure umfasst.

Wie schon bei den zuvor beschriebenen Immobilisierungs- und Anreicherungsverfahren steht die Reihenfolge, in der die Schritte a) und b) durchgeführt werden, im Belieben des Fachmanns. Dieser kann anhand der Umstände des Einzelfalles leicht selbst entscheiden, welche Reihenfolge die besten Nachweisergebnisse liefert.

Zweckmäßigerweise wird das Nachweisverfahren, und/oder in entsprechender Weise das Immobilisierungs- bzw. Anreicherungsverfahren, in Form eines miniaturisierten Assays durchgeführt. Beispielsweise können verschiedene zu untersuchenden Proben jeweils in eine Vertiefung einer Mikrotiterplatte gegeben und dort einem für die jeweilige Vertiefung spezifisch ausgestalteten Nachweisverfahren unterzogen werden. Auf diese Weise können große Probenmengen parallel verarbeitet werden. Ebenfalls ist es möglich, verschiede Mikroorganismen parallel zueinander nachzuweisen, indem in den einzelnen Vertiefungen verschiedene, für den jeweiligen Mikroorganismus spezifische Nachweisreagenzien verwendet werden. Zur Herstellung von Peptidarrays im allgemeinen kann sich der Fachmann beispielsweise orientieren an Current Protocols in Cell Biology, Kap. 9.8: Identification of antigenic determinants using synthetic peptide combinatorial libraries, John Wiley & Sons.

Ebenfalls besonders bevorzugt ist es, wenn der nachzuweisende Mikroorganismus vor dem Nachweisen des Markers mit einem der zuvor beschriebenen erfindungsgemäßen Immobilisierungs- und/oder Anreicherungsverfahren angereichert wird. Auf diese Weise kann der nachzuweisende Mikroorganismus ohne großen Aufwand auch in stark verdünnten Proben oder in Proben mit den Nachweis stark störenden Begleitsubstanzen zuverlässig und mit hoher Sensitivität nachgewiesen werden.

Erfindungsgemäß ist es besonders vorteilhaft, zum Nachweisen von *M. paratuberculosis* ein Nachweisverfahren gemäß der zuvor beschriebenen Art durchzuführen, bei dem ein Nachweisreagens verwendet wird, das ein Bindungspeptid wie eingangs beschrieben umfasst.

Die erfindungsgemäßen Bindungspeptide besitzen eine hohe Spezifität und Affinität für *M. paratuberculosis,* so dass sie sich besonders zum Nachweisen dieses Mikroorganismus eignen.

Ferner wird ein Kit zum Anreichern von Mikroorganismen bereitgestellt, umfassend:
a) ein Fängerpeptid und/oder ein Bindungspeptid, jeweils wie zuvor beschrieben, und
b) einen festen Träger.

Mit einem solchen Kit wird dem Fachmann auf einfache Weise eine Reagenzienzusammenstellung an die Hand gegeben, um ein erfindungsgemäßes Fängerreagens leicht selbst herzustellen bzw. die Bindung eines Bindungspeptids an einen festen Träger ohne großen Aufwand zu ermöglichen. Damit vereinfacht ein solcher Kit die Durchführung eines erfindungsgemäßen lmmobilisierungs- bzw. Anreicherungsverfahrens. Partikelförmige feste Träger sind wiederum wegen der genannten Vorteile besonders bevorzugt.

In entsprechender Weise wir auch ein Kit zum Nachweisen von Mikroorganismen bereitgestellt, umfassend:
a) ein Fängerpeptid und/oder ein Bindungspeptid, jeweils wie zuvor beschrieben, und
b) einen Marker.

Eine solche Reagenzienzusammenstellung vereinfacht die Herstellung eines erfindungsgemäßen Nachweisreagenzes.

Zum Nachweisen von *M. paratuberculosis* ist ein Kit bevorzugt, der umfasst::
a) ein Fängerpeptid und/oder ein Bindungspeptid, jeweils wie zuvor beschrieben, und
b) eine oder mehrere Nucleinsäuren gemäß SEQ ID NO. 10 und/oder SEQ ID NO. 11.

Die Nucleinsäuren gemäß SEQ ID NO. 10 und SEQ ID NO. 11 eignen sich auf vorteilhafte Weise dazu, spezifisch an das Genom von *M. paratuberculosis* zu binden und als PCR-Primer zum spezifischen Vervielfältigen eines Abschnitts des Genoms von *M. paratuberculosis* eingesetzt zu werden. Ein Beispiel zur Verwendungsweise der Nucleinsäuren gemäß SEQ ID NO. 10 und SEQ ID NO. 11 ist nachfolgend angegeben. Für weitere Details zur Verwendung der genannten Nucleinsäuren wird der Fachmann auch die Veröffentlichung von B. Strommenger et al., Isolation and diagnostic potential of ISMAV2, a novel insertion sequence like element from *Mycobacterium* *avium* subspecies *paratuberculosis,* FEMS Microbiology Letters 196 (2001), 31-37, zu Rate ziehen.

Die Erfindung betrifft gemäß einem weiteren Aspekt auch Verfahren zur Herstellung der Fänger- bzw. Bndungspeptide. In diesem Zusammenhang wird ein Waschpuffer angegeben, umfassend:
a) 2-6M Guanidinhydrochlorid und/oder 4-8M Harnstoff, und
b) 50 mM Tris-HCl (pH 7,5), 150 mM NaCI und 0,05 % (v/v) Tween-20.

Dieser Puffer ermöglicht ein besonders stringentes Waschen von an die nachzuweisenden Mikroorganismen gebundenen Peptiden bei der Herstellung der erfindungsgemäßen Fängerpeptide bzw. Bindungspeptide.

Die Erfindung betrifft auch allgemein ein Verfahren zum Bereitstellen einer Nucleinsäure, die einen für ein Peptid codierenden Abschnitt umfasst, wobei das von diesem Abschnitt codierte Peptid an eine Zielsubstanz binden kann, umfassend die Schritte:
a) Bereitstellen verschiedener Peptide, die jeweils mit einer zugeordneten Nucleinsäure verbunden sind, wobei die Nucleinsäure einen für das jeweilige Peptid codierenden Abschnitt besitzt,
b) Inkontaktbringen der Zielsubstanz mit den verschiedenen Peptiden,
c) Abtrennen von nicht an die Zielsubstanz gebundenen Peptiden, wobei zum Abtrennen ein Waschpuffer der soeben beschriebenen Art verwendet wird, und
d) Aufreinigen einer Nucleinsäure, die mit einem an die Zielsubstanz gebundenen Peptid verbunden ist.

In einem solchen Bereitstellungsverfahren ist ein besonders stringentes Waschen erforderlich, um solche Peptide, die an die Zielsubstanz bindend, von solchen mit geringerer Affinität abzutrennen. Ohne ein stringentes Waschen in Schritt c) werden zwar eine Vielzahl verschiedener Nucleinsäuren in Schritt d) aufgereinigt, nur verhältnismäßig wenige dieser aufgereinigten Nucleinsäuren werden jedoch für ein Peptid mit hoher Affinität und Spezifität für die Zielsubstanz codieren. Zweckmäßigerweise werden die Schritte a) bis d) mehrfach wiederholt, so dass die jeweils in Schritt d) aufgereinigten Nucleinsäuren und Peptide erneut mit der Zielsubstanz in Kontakt gebracht werden, um weniger spezifisch und/oder affin an die Zielsubstanz bindende Peptide von stärker spezifischen und/oder affinen Peptiden abzutrennen. Es wird so auf vorteilhaft einfache Weise erreicht, dass mit jedem Durchlaufen der Schritte a) bis d) stärker spezifische und/oder höher affinere Peptide als zuvor erhalten werden.

Es ist nun ein solches Verfahren besonders bevorzugt, bei dem die Peptide und die zugeordneten Nucleinsäuren als Phagen-Display-Bank vorliegen. Phagen-Display-Banken sind dem Fachmann bereits bekannt, beispielsweise wird von New England Biolabs ein Ph.D.-12 genannter Phage Display Peptide Library Kit angeboten. Dieser umfasst eine kombinatorische Bibliothek zufälliger 12-mer-Peptide, die an ein kleines Hüllprotein (minor coat protein pIII) des M13-Phagen fusioniert sind. Die 12-mer-Peptide werden am N-Terminus des pIII-Genprodukts exprimiert, gefolgt von einer kurzen Spacer-Sequenz (Gly-Gly-Gly-Ser) und der Wildtyp-pIII-Sequenz. Bei der Durchmusterung (Biopanning) einer solchen Phagen-Bibliothek müssen die an die Zielsubstanz gebundenen Phagen zwar sorgfältig von den nicht gebundenen Phagen getrennt werden. Von der Verwendung chaotroper Reagenzien wie Guanidinhydrochlorid und Harnstoff wird jedoch herkömmlicherweise ausdrücklich abgeraten, da die Phagen nach einer Behandlung mit diesen Reagenzien nicht mehr infektös seien und somit keine oder nur eine sehr schlechte Vermehrung der selektierten Phagen möglich sei. Ganz im Gegensatz zu diesem Urteil der Fachwelt wurde nunmehr gefunden, dass ein Waschpuffer der beschriebenen Art keine nennenswerte Einbuße hinsichtlich der Infektiosität der Phagen bewirkt, sondern zu einer hoch stringenten Selektion der an die Zielsubstanz bindenden Phagen führt. Damit ist es erstmals möglich, ein Biopanning unter hoch stringenten Bedingungen durchzuführen, um Phagen zu selektieren, die Peptide exprimieren, die spezifisch an eine Zielsubstanz binden können.

Besonders vorteilhaft ist ein solches Biopanning mit einer Phagen-Display-Bank zur Selektion von Phagen, die Peptide exprimieren, die spezifisch an *M. paratuberculosis* binden können.

Schließlich werden noch erfindungsgemäß Nucleinsäuren mit einem für ein Peptid codierenden Abschnitt angegeben, wobei das Peptid ein Fängerund/oder Bindungspeptid der zuvor beschriebenen Art ist. Solche Nucleinsäuren gestatten es, auf einfache Weise Fänger- und/oder Bindungspeptide herzustellen.

Vorzugsweise umfasst jede der Nucleinsäuren einen Ausschnitt mit einer Sequenz gemäß einer der Sequenzen SEQ ID 12 bis SEQ ID 62. Dabei bedeuten die Nucleotidsymbole gemäß dem fachüblichen Abkürzungsstandard:
n: a,c,g oder t
h: a, c oder t
r: a oder g
y: c oder t.

Die Nucleinsäuren gemäß SEQ ID NO. 12 und SEQ ID NO. 21 codieren dabei für ein Peptid mit der Aminosäuresequenz SEQ ID NO. 1, die Nucleinsäuren gemäß SEQ ID NO. 13 und SEQ ID NO. 22 codieren für ein Peptid mit der Aminosäuresequenz SEQ ID NO. 2 und so weiter. Soll die Aminosäuresequenz des Peptids nicht die gesamte Aminosäuresequenz des Peptids gemäß einer der Sequenzen SEQ ID NO. 1 bis SEQ ID NO. 9 umfassen, sondern lediglich einen kürzeren Ausschnitt eines solchen Peptids, so kann der Fachmann die entsprechenden Codons aus den Nucleinsäuren gemäß SEQ ID NO. 12 bis SEQ ID NO. 29 auswählen. Entsprechendes gilt für die Sequenzen SEQ ID NO. 30 bis SEQ ID NO. 62; diese codieren für die Peptide SEQ ID NO. 63 bis SEQ ID NO. 95.

Besonders bevorzugt sind solche Nucleinsäuren, die es ermöglichen, das Peptid stark und in einer leicht aufzureinigenden Weise zu exprimieren. Dabei kann das Peptid ein Teil eines größeren Moleküls sein, beispielsweise in Form eines Fusionsproteins. In besonders bevorzugten Nucleinsäuren ist der Abschnitt, der für das Peptid codiert, in einem Sequenzabschnitt angeordnet, der für ein abspaltbares Signalpeptid codiert. Beispielsweise kann eine solche Nucleinsäure mit Hilfe eines pMAL-Fusionsprotein-Systems (New England Biolabs) leicht hergestellt werden. In anderen bevorzugten Nucleinsäuren ist der Abschnitt, der für das Peptid codiert, in einem Gen für Klebsiella-Typ 3-Fimbrien angeordnet. Diese werden bei Erwärmung auf 45 °C abgeworfen, so dass Fusionsproteine leicht präpariert werden können.

Die Erfindung wird nachfolgend anhand der Beispiele näher beschrieben. Soweit nichts anderes angegeben wird, sind alle Pufferzusammensetzungen die in Sambrook et al., Molecular Cloning, 3. Auflage 2001, angegebenen.

### Beispiel 1: Herstellen bakterieller Kulturen

*Mycobacterium avium* subspezies *paratuberculosis* wurde auf Middlebrook 7H10-Agar (Difco Laboratories, Detroit, Ml, USA), angereichert mit 100 ml OADC (pro Liter: 0,85 g NaCl, 0,5 g Rinderserumalbumin (BSA) Fraktion V, 20 g Dextrose, 3 mg Katalase, 60 µl Ölsäure, 0,2 % Glycerin, 2 µg/ml Mycobactin (Symbiotics, Lyon, Frankreich)) kultiviert. Für die weitere Verwendung wurden die Mycobacterien vorsichtig vom Agar entnommen und in geeigneten Puffern resuspendiert. Die Suspension wurde durch 5 minütiges Vortexen mit Glas-Beads (pro 5 ml bakterieller Suspension: 30 Beads mit je 2 mm Durchmesser) in einem Polypropylen-Gefäß homogenisiert. *Escherichia coli*-Stämme wurden auf Luria-Bertani-(LB)-Medium inkubiert; zum Ausplattieren von Phagen wurden lsopropylthiogalaktosid (IPTG, 1 mM Endkonzentration) und X-Gal (60 mM Endkonzentration) zur Top-Agarose zugegeben.

### Beispiel 2: Selektion von Bacteriophagen - Biopanning

Zum Biopanning wurde ein Ph.D.-12 Phage Display Peptide Library Kit (New England Biolabs, Beverly, MA, USA) verwendet. Die Bibliothek (Library) umfasst zufällige 12-mer Peptide; sie besitzt eine Komplexität von 1,9 × 10⁹ und weist eine Konzentration von 3,8 × 10¹² Plaque-bildenden Einheiten pro Milliliter (pfu/ml) auf.

Im ersten Biopanning-Durchgang wurden 10 µl der Bibliothek (3,8 × 10¹⁰ pfu/ml) mit 1 ml des *M. paratuberculosis*-Stammes 6783 (OD660 = 0,5) eine Stunde lang bei Raumtemperatur inkubiert. Die Mycobacterien wurden acht Mal mit TBST-Puffer (50 mM Tris-HCI (pH 7,5), 150 mM NaCI, 0,05% (v/v) Tween-20) unter Zentrifugation gewaschen. Die Phagen wurden mit einem Glycin-BSA-Puffer (0,2 M Glycin-HCI (pH 2,2), 1 mg/ml BSA) eluiert. Das Eluat wurde sofort mit Tris-HCl-Puffer (1 M Tris-HCI, pH 9,1) neutralisiert. Der Phagentiter wurde durch serielle Verdünnung um jeweils den Faktor 10 und Ausplattieren auf einer Kultur des *E.* coli-Stammes Top 10 F' (Invitrogen Co., Carlsbad, CA, USA) bestimmt.

Die Phagen wurden für den zweiten Biopanning-Durchgang auf dem gleichen *E. coli*-Stamm vermehrt. Die vermehrten Phagen wurden mit einem wässrigen Puffer aus Polyethylenglycol (20 %) und NaCI (2,5 M) präzipitiert und in PBS (phosphate buffered saline, 150 mM NaCI, 1,5 mM KH₂PO₄, 9 mM Na₂HPO₄, 2,5 mM KCI, pH 7,2) resuspendiert.

In den nachfolgenden Biopanning-Durchgängen wurden durchschnittlich 3,75 × 10¹¹ pfu der vermehrten Phagen eingesetzt Fünf Biopanning-Durchgänge wurden durchgeführt, im letzten Durchgang enthielt der zum Waschen verwendete TBST-Puffer zusätzlich entweder Guanidinhydrochlorid (4 M) oder Harnstoff (6 M).

Nach dem letzten Biopanning-Durchgang wurden 30 einzelne Plaques gepickt, die Peptid-spezifische DNA wurde mittels PCR amplifiziert (Primer - 96M13glll und +130M13glll, New England Biolabs) und anschließend sequenziert.

### Beispiel 3: Markieren der Phagen mit Fluorescein-Isothiocyanat (FITC)

Ausgewählte Phagen wurden vermehrt (Phagen-Klone), präzipitiert und in PBS auf eine Endkonzentration von 10¹² pfu/ml resuspendiert.

Zum Markieren der Phagen mit Fluoreszein-lsothiocyanat (FITC, Pierce Inc., Rockford, IL, USA) wurden 500 µl eines Carbonatpuffers (50 mM Carbonat, pH 9,8), 1 ml PBS und 400 µl FITC (1 mg/ml FITC, in PBS) zu 500 µl der Phagensuspension (10¹² pfu/ml) gegeben und über Nacht bei 4°C inkubiert. Ungebundenes FITC wurde durch Präzipitieren der Phagen, wie in Beispiel 2 beschrieben, entfernt.

### Beispiel 4: Markieren der Phagen mit NHS-LC-Biotin

Zum Markieren von Phagen mit NHS-LC-Biotin (Molecular Biosciences Inc., CO, USA) wurde zunächst wie in Beispiel 3 beschrieben eine Phagensuspension mit einer Endkonzentration von 10¹² pfu/ml hergestellt.

Zu 100 µl der Phagensuspension wurden 10 µl NHS-LC-Biotin (5 mg/ml, in DMSO) zugegeben und 1h lang auf Eis inkubiert. Ungebundenes NHS-LC-Biotin wurde durch Präzipitieren der Phagen, wie in Beispiel 2 beschrieben, entfernt.

### Beispiel 5: Markieren der Phagen mit Alkalischer Phosphatase

Biotinylierte Phagen wurden 1 h lang mit Streptavidin-Alkalischer Phosphatase (20 µg/ml) bei Raumtemperatur inkubiert. Ungebundenes Enzym wurde durch 15minütige Dialyse auf schwimmenden Membranen mit einem Porendurchmesser von 0,025 µm (Millipore, Eschborn, Deutschland) abgetrennt.

### Beispiel 6: Binden der Phagen an paramagnetische Partikel

Zum Binden der Phagen an paramagnetische Partikel wurden 100 µg Streptavidin-beschichteter paramagnetischer MagneSphere-Partikel (Promega Inc., Madison, Wl., USA) mit einer Phagen-Suspension (0,5 × 10¹² pfu in PBS) 6 h lang bei 4 °C inkubiert. Um ungebundene Phagen zu entfernen wurden die Partikel drei Mal mit PBS gewaschen.

### Beispiel 7: Platten-Bindungs-Assay

Es wurde eine *M. paratuberculosis*-Suspension in Carbonatpuffer (35 mM NaHCO₃, 15 mM Na₂CO₃, pH 9,8) hergestellt. Die OD660 der Suspension wurde auf 1,0 eingestellt, dies entspricht etwa 10⁸ Bakterien pro Milliliter. Maxisorp-Mikrotiterplatten (Nunc, Roskilde, Dänemark) wurden mit dieser *vorbereiteten Suspension beschichtet, indem 100 µl der bakteriellen Suspension in jede Vertiefung pipettiert und darin über Nacht bei 4 °C inkubiert wurden. Zum Blockieren über Nacht bei 4 °C wurde ein Gelatine-(0,5 %)-haltiger Überstand einer *E. coli* Top 10 F'-Kultur, die mit der gesamten Phagen-Bibliothek infiziert worden war, verwendet.

Um das Binden von mit Alkalischer Phosphatase verbundenen Phagen an verschiedene Stämme von *M. paratuberculosis* und einen Referenzstamm von *M. avium* zu überprüfen, wurden serielle Verdünnungsstufen mit jeweils dem Verdünnungsfaktor 2 der einzelnen Phagen-Klone 1h lang bei Raumtemperatur in den entsprechend beschichteten Mikrotiterplatten (wie vorstehend beschrieben) inkubiert. Die Mikrotiterplatten wurden gewaschen mit einem Puffer aus 0,1fach PBS und 0,05 % Tween-20, und mit p-Nitrophenylphosphat (pNPP) entwickelt.

### Beispiel 8: Synthese künstlicher Peptide

Künstliche Peptide wurden auf der Grundlage der Sequenz (Beispiel 2) der selektierten Phagen nach üblichen Verfahren unter N-terminaler Biotinylierung hergestellt.

### Beispiel 9: Binden der Peptide an Streptavidin-beschichtete paramagnetische Partikel

Es wurden 100 µg Streptavidin-beschichteter paramagnetischer MagneSphere-Partikel (Promega Inc.,Madison, Wl, USA) mit 20 µg des biotinylierten Peptids in PBS 6 h lang bei 4 °C inkubiert. Die Partikel wurden anschließend drei Mal mit PBS gewaschen.

### Beispiel 10: Anreicherung von M. paratuberculosis aus Milch

Es wurde 1 ml Milch natürlich infizierter Kühe oder künstlich kontaminierter ganzer Milch mit 10 µl der beschichteten paramagnetischen Partikel (Beispiele 6. 9) bei 4°C unter sanftem Schwenken inkubiert. Die Partikel wurden wie vom Hersteller empfohlen reisoliert und drei Mal mit einem Puffer gewaschen, der aus 0,1fach PBS und 0,05 % (v/v) Tween-20 bestand.

### Beispiel 11: Bestätigung der Bindung von M. paratuberculosis an paramagnetische Partikel - Acridinorange-Färbung

Zum Nachweis der Bindung von *M. paratuberculosis* an die paramagnetischen Partikel (Beispiele 6, 9) wurden 5 µl der Suspension aus Beispiel 10 auf einen Mikroskopie-Objektträger überführt, luftgetrocknet und hitzefixiert. Der Objektträger wurde mit einer 0,1 %igen (w/v) Acridinorange-Lösung, die 0,6 % (v/v) Phenol enthielt, überschichtet und 15 Minuten lang inkubiert. Die Objektträger wurden mit Wasser gewaschen und getrocknet. Zur Gegenfärbung und zum Entfärben wurde der Objektträger 2 Minuten lang mit einer sauren Alkohollösung, umfassend 0,2 % (w/v) Methylenblau 2, inkubiert. Die Objektträger wurden mit einem Fluoreszenzmikroskop ausgewertet, säurefeste Bakterien waren hell orange gegen einen dunklen Hintergrund gefärbt, während die paramagnetischen Partikel keine Fluoreszenz aufwiesen.

### Beispiel 12: Behandlung gebundener Mycobacterien für die PCR

Nach dem Reisolieren und Waschen (Beispiel 10) wurden die paramagnetischen Partikel in 50 µl 0,1fach TE-Puffer (1fach TE: 10 mM Tris-HCI (pH 8,0), 1 mM EDTA) resuspendiert und in einem Mikrowellenherd 8 min lang bei 180 Watt gekocht. Die mycobacterielle DNA wurde mit einem Gene clean-Kit (Qbiogene Inc, Carlsbad, CA, USA) nach den Herstellerangaben isoliert und in 25 µl destilliertem Wasser aufgenommen.

### Beispiel 13: Nachweis von M. paratuberculosis durch PCR

Für die PCR wurden 5 µl der DNA-Lösung aus Beispiel 12 eingesetzt und mit 20 µl eines PCR-Mastermixes gemischt. Der PCR-Mastermix bestand aus (Konzentrationen des unverdünnten Mastermixes):

| | |
|---|---|
| MgCl₂ | 1,5 mM |
| | |
| PCR Puffer (Invitrogen Co., Carlsbad, CA. USA) | 1x |
| | |
| dATP, dCTP, dGTP, dTTP, jeweils | 0,2 mM |
| Primer (SEQ ID 10, SEQ ID 11), jeweils | 0,5 µM |
| | |
| Taq DNA-Polymerase (Invitrogen Co., Carlsbad, CA, USA) | 0,02 U/µl |

Die PCR wurde unter folgenden Bedingungen durchgeführt:

| | | |
|---|---|---|
| 1. | Denaturierung: | keine Wiederholung |
| | 3 min 94 °C | |
| | | |
| 2. | Vervielfältigung: | 32 Wiederholungen |
| | 30 sek 94 °C | |
| | 30 sek 64,5 °C | |
| | 30 sek 72°C | |
| | | |
| 3. | Auffüllen (Extension): | keine Wiederholung |
| | 10 min 72°C | |

### Beispiel 14: Bestimmen der Nachweisgrenze

Es wurde eine serielle Verdünnungsreihe mit dem Verdünnungsfaktor 10 pro Verdünnungsstufe aus künstlich kontaminierter Milch (in der unverdünnten Konzentration 10⁸ cfu *M. paratuberculosis* pro ml, f.c., überprüft durch Zählung in einer Thoma-Kammer) hergestellt.

Jeweils 1 ml der kontaminierten Milch mit 10⁵ cfu *M. paratuberculosis*/ml bis 10⁰ cfu *M. paratuberculosis*/ml wurden mit 10 µl der beschichteten paramagnetischen Partikel (Beispiele 6, 9) inkubiert. Anschließend wurde wie in den Beispielen 10, 12 bis 13 beschrieben verfahren.

Mit den Peptiden der SEQ ID NO. 3 und SEQ ID NO. 8 konnten 1-10 cfu *M. paratuberculosis*/ml kontaminierter Milch nachgewiesen werden.

### Beispiel 15: Isolierung rekombinanter M13-Phagen, die spezifisch sind für ein mutmaßliches Outer-Membrane-Protein von M. paratuberculosis und seine anfängliche Charakterisierung

Das *M. paratuberculosis*-Transportersystem (Mpt) wurde auf Basis einer "representational difference"-Analyse als mutmaßlicher ABC-Transporter (Strommenger, 2001) identifiziert. Die offenen Leserahmen des mpt-Genclusters mptA bis mptF (Gene bank accession number AF 419325) wurden ihrer Sequenz nach und mit einer TMHMM-Analyse analysiert. Das mutmaßliche MptD-Protein zeigte keine signifikanten Homologien zu bekannten Proteinen. Die TMHMM-Analyse legte jedoch nahe, dass es sechs Transmembran-Helices umfasse und dass es ein kleineres Outer-Membrane-Protein sei.

Ein Teil des Mpt-Genclusters, einschließlich des offenen Leserahmens mptD wurde daraufhin in *M. smegmatis* mc²155 und *M. avium* DSM 14557 mit Hilfe des integrierenden mycobacteriellen Vektors pMV 361 (Strommenger, 2001) kloniert. Eine Expression des mutmaßlichen MptD-Proteins gelang jedoch nicht in *E. coli,* Antikörper konnten nicht gewonnen werden.

Um nachzuweisen, dass MptD exprimiert wird und in der äußeren Membran lokalisiert ist, wurden zwei verschiedene Versuchswege beschritten. Die transformierten *M. smegmatis* mc²155-Bakterien, die den offenen Leserahmen mptD beinhalteten, wurden als Grundlage für ein Biopanning der Phagen-Bibliothek verwendet (s. Beispiel 2 ff). Die so erhaltenen spezifisch bindenden Phagen sollten die Charakterisierung von MptD ermöglichen und dazu verwendet werden, die Expression von MptD in vitro und in vivo nachzuweisen, um die Expression und Funktion des mpt-Operons zu klären.

Um rekombinante Phagen zu isolieren, die spezifisch für MptD aus *M. paratuberculosis* sind, wurden in beiden Versuchswegen *M. smegmatis* pIIID320-Transformanten verwendet, die den offenen Leserahmen mptD aus *M. paratuberculosis,* einkloniert in den mycobacteriellen Vektor pMV 361, beinhalteten. Im ersten Versuchsweg wurde ein Biopanning mit vollständigen *M. smegmatis*-Transformanten durchgeführt, im zweiten Versuchsweg wurde ein Biopanning im wesentlichen nur mit den Membranen der *M. smegmatis*-Transformanten durchgeführt. Beide Versuchswege ergaben einen dominierenden, für MptD spezifischen Phagen, dessen relevante Bindungssequenz fMptD1 (SEQ ID NO 30) genannt wurde.

### Beispiel 16: Bestätigung der Anwesenheit des mptD-offenen Leserahmens in M. smegmatis- und M. avium-Transformanten

Um zu überprüfen, ob die *M. smegmatis-* und *M.* avium-Transformanten (*M. smegmatis* pRD320 und *M. avium* pRD320) den mptD-offenen Leserahmen umfassten wurde eine PCR zum einen mit internen Primern durchgeführt, die spezifisch für das klonierte Fragment waren, und zum anderen mit Primern gegen den Kanamycin-Resistenzmarker, der im mycobacteriellen Vektor pMV361 codiert ist. Mit diesem Vektor waren *M. smegmatis* und *M. avium* transformiert worden. Die PCR mit den internen Primern lP30 und lP31 ergab ein Produkt von 247 Basenpaaren Länge bei gekochtem Koloniematerial sowohl von M. paratuberculosis als auch von den *M. smegmatis* pRD320 und *M. avium* pRD320-Transformanten. Kein PCR-Produkt wurde erhalten, wenn Koloniematerial von *M. smegmatis* und *M. avium* verwendet wurde, die den pMV361-Vektor ohne das klonierte Fragment beinhalteten. Primer gegen den Kanamycin-Resistenzmarker des pMV361-Vektors erzeugten ein PCR-produkt von 590 Basenpaaren Länge mit allen *M. smegmatis-* und *M. avium*-Transformanten, nicht jedoch mit gekochtem Koloniematerial von *M. paratuberculosis* 6783. Diese Befunde zeigen, dass für das Biopanning nur Transformanten mit dem offenen Leserahmen für mptD verwendet wurden.

### Beispiel 17: Isolierung rekombinanter M13-Phagen. die spezifisch für MptD sind, mittels M. smegmatis-Transformanten, die den mptD-offenen Leserahmen beinhalten

Zum Isolieren spezifischer rekombinanter Phagen gegen das Transmembranprotein MptD wurde das Biopanning-Verfahren modifiziert. Subtraktionsschritte wurden eingefügt, um Phagen zu eliminieren, die an andere Oberflächenstrukturen der *M. smegmatis*-Transformanten, die den Vektor pMV361 enthalten, binden.

Zur Subtraktion wurden *M. smegmatis*-Transformanten verwendet, die nur den Vektor pMV361 enthielten. Im letzten Biopanning-Schritt wurde ein hochstringenter Waschschritt mit einem chaotrophem Puffer durchgeführt (s.o.).

Nach 5 Biopanning-Runden wurden 48 einzelne Plaques gepickt und sequenziert. Zum Sequenzieren wurden die Primer +130M13 und -96M13 verwendet. Das Sequenzieren ergab 13 unterschiedliche Sequenzen, wobei einzelne Sequenzen 9 bis 10 Mal vorkamen. Nicht alle Plaques konnten sequenziert werden. Jede Sequenz codiert für ein einzigartiges Polypeptid, das im minor phage pIII-Hüllportein exprimiert wird. Die Sequenzierung offenbarte eine dominierende Sequenz und dementsprechen ein dominierendes rekombinantes 12-mer Peptid, das im minor phage pIII-Hüllprotein exprimiert wird. Der zugehörige MptD-spezifische Phage bzw. dessen für das Peptid codierende Sequenz wurde fMptD1 genannt (SEQ ID NO 30), die davon abgeleitete Peptidsequenz wurde aMptD1 genannt (SEQ ID NO 63).

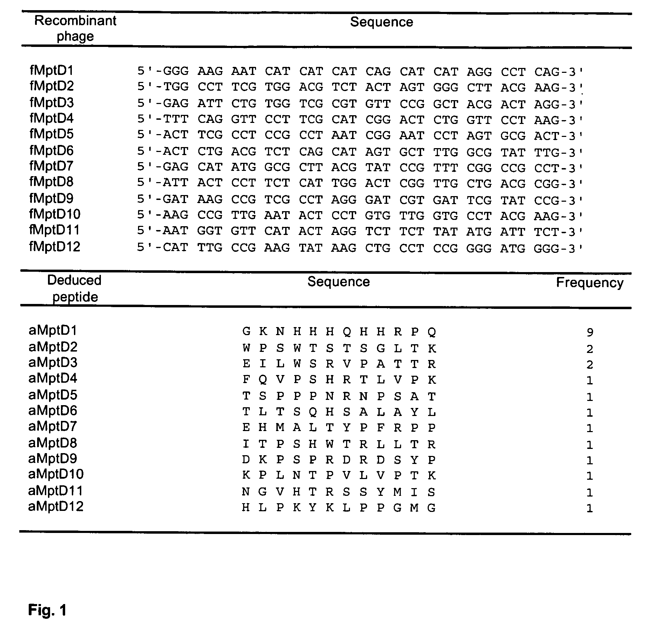

Figur 1 zeigt die durch Sequenzierung erhaltenen Nucleinsäure-Sequenzen, die daraus abgeleiteten Aminosäure-Sequenzen sowie die Häufigkeit, mit der die jeweilige Nucleinsäure-Sequenz gefunden wurde.

### Beispiel 18: Isolierung rekombinanter M13-Phagen, die spezifisch für MptD sind. mittels Membranfraktionen von M. smegmatis-Transformanten, die den mptD-offenen Leserahmen beinhalten

In diesem Versuchsweg wurde das Biopanning mit Membranpräparationen der *M. smegmatis* pRD320-Transformanten durchgeführt, die den mptD-offenen Leserahmen beinhalteten. Die Membranpräparationen wurden an CNBr-aktivierte Sepharose gekoppelt und das soeben beschriebene modifizierte Biopanning wurde durchgeführt.

Zur Subtraktion wurden an CNBr-aktiviere Sepharose gekoppelte Membranpräparationen von *M. smegmatis*-Transformanten verwendet, die nur den Vektor pMV361 beinhalteten. Der letzte Biopanning-Schritt wurde mit einem hochstringenten Waschschritt durchgeführt, in dem ein Tris-gepufferter Tween-Puffer mit Guanidinhydrochlorid verwendet wurde (s.o.).

Nach 5 Biopanning-Runden wurden 48 einzelne Plaques gepickt und sequenziert. Das Sequenzieren wurde wie soeben beschrieben durchgeführt. Es ergab 22 verschiedene Sequenzen, die jeweils für ein einzigartiges Polypeptid codierten, das im minor phage pIII-Hüllprotein exprimiert wird. Einzelne Sequenzen traten bis zu 8 Mal auf. Nicht alle Plaques konnten sequenziert werden. Die Sequenzierung offenbarte eine dominierende Sequenz und dementsprechen ein dominierendes rekombinantes 12-mer Peptid, das im minor phage pIII-Hüllprotein exprimiert wird. Diese Sequenz trat auch in dem Biopanning gemäß dem vorherigen Beispiel auf. Dementsprechend wurde der zugehörige MptD-spezifische Phage bzw. dessen für das Peptid codierende Sequenz fMptD1 genannt (SEQ ID NO 30), die davon abgeleitete Peptidsequenz wurde aMptD1 genannt (SEQ ID NO 63).

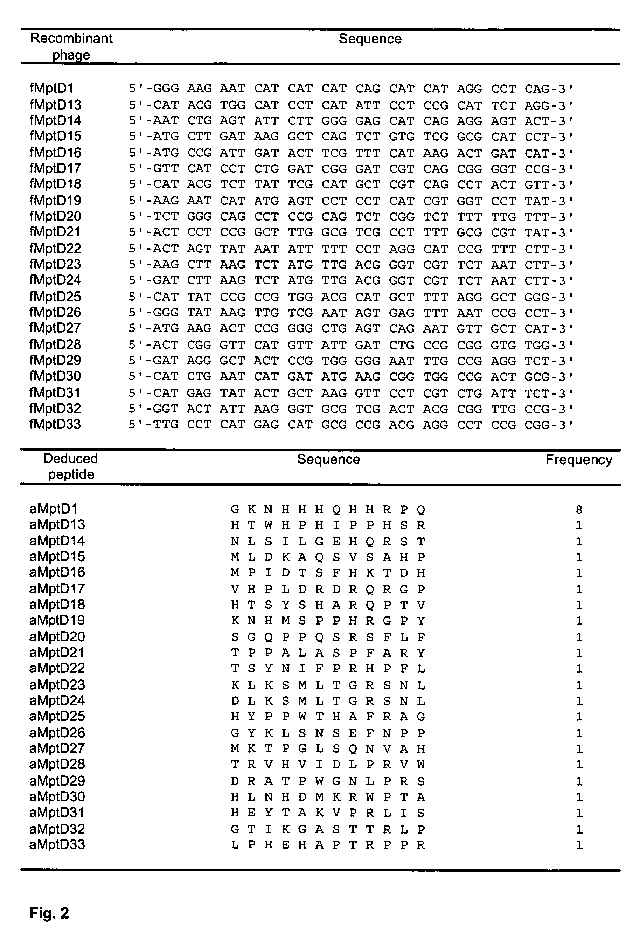

Figur 2 zeigt die durch Sequenzierung erhaltenen Nucleinsäure-Sequenzen, die daraus abgeleiteten Aminosäure-Sequenzen sowie die Häufigkeit, mit der die jeweilige Nucleinsäure-Sequenz gefunden wurde.

### Beispiel 19: Demonstration der M. paratuberculosis-Expression von MptD in infizierten Maus-Makrophagen

J774 Maus-Makrophagen wurden auf Deckgläschen kultiviert und mit lebenden, in Flüssigmedium kultivierten *M. paratuberculosis* infiziert. Um eine erfolgreiche Infektion der J774 Maus-Makrophagen nachzuweisen wurde eine Ziehl-Neelsen-Färbung nach der Infektion durchgeführt. Es konnte nachgewiesen werden, dass eine Phagocytose von *M. paratuberculosis* stattgefunden hatte.

Zwei Stunden nach der Infektion wurden die Zellen mit FITC-markierten fMptD-Phagen inkubiert. Die ursprüngliche Phagen-Bibliothek wurde ebenfalls mit FITC markiert und diente als negative Kontrolle. Nach der Inkubation mit den Phagen wurden die Zellen gewaschen, getrocknet und fluoreszenzmikroskopisch untersucht.

*M. paratuberculosis* konnte in Zellen nachgewiesen werden, die mit FITC-markierten fMptD-Phagen inkubiert worden waren. Der Nachweis von *M. paratuberculosis* gelang nicht mit den FITC-markierten Kontrollphagen.

Diese Befunde lassen vermuten, dass MptD von *M. paratuberculosis* in vitro in Makrophagen exprimiert wird.

## Patentansprüche

1. Bindungspeptid zum Binden an *Mycobacterium paratuberculosis,* umfassend
a) einen 8 Aminosäuren langen Ausschnitt aus einer Aminosäuresequenz gemäß einer der Sequenzen SEQ ID NO. 1 bis SEQ ID NO. 9 oder SEQ ID NO. 63 bis SEQ ID NO. 95 oder
b) einen zu einem Ausschnitt gemäß a) funktionsgleichen, 8 Aminosäuren langen Ausschnitt.

2. Bindungspeptid nach Anspruch 1, **dadurch gekennzeichnet, dass** die Aminosäuresequenz des Bindungspeptids die Aminosäuresequenz gemäß SEQ ID NO. 3, SEQ ID NO. 8 oder SEQ ID NO. 63 ist.

3. Anreicherungsverfahren zum Anreichern von *Mycobacterium paratuberculosis,* umfassend die Schritte:
a) Binden eines Bindungspeptids nach einem der Ansprüche 1 bis 2 an einen festen Träger, und
b) Inkontaktbringen des Bindungspeptids mit einer zu untersuchenden Probe, um ein Binden des Bindungspeptids an *Mycobacterium paratuberculosis* zu ermöglichen.

4. Anreicherungsverfahren nach Anspruch 3, **dadurch gekennzeichnet, dass** Schritt a) vor Schritt b) durchgeführt wird.

5. Nachweisreagens zum Nachweisen eines Mikroorganismus, umfassend
a) ein Bindungspeptid nach einem der Ansprüche 1 bis 2, verbunden mit
b) einem Marker.

6. Nachweisreagens nach Anspruch 5, **dadurch gekennzeichnet, dass** der Marker radioaktiv, fluoreszierend, phosphoreszierend und/oder farbig ist und/oder eine Nucleinsäure und/oder eine katalytisch wirkende Einheit umfasst.

7. Nachweisverfahren zum Nachweisen eines Mikroorganismus, umfassend die Schritte:
a) Inkontaktbringen einer zu untersuchenden Probe mit einem Nachweisreagens nach einem der Ansprüche 5 bis 6, um ein Binden des Bindungspeptids an den nachzuweisenden Mikroorganismus zu ermöglichen, und
b) Nachweisen des Markers des Nachweisreagens.

8. Nachweisverfahren nach Anspruch 7, **dadurch gekennzeichnet, dass** der nachzuweisende Mikroorganismus vor dem Nachweisen des Markers mit einem Verfahren einem der Ansprüche 3 bis 4 angereichert wird.

9. Nachweisverfahren zum Nachweisen von *Mycobacterium paratuberculosis,* **dadurch gekennzeichnet, dass** ein Nachweisverfahren nach einem der Ansprüche 7 bis 8 durchgeführt wird, bei dem ein Nachweisreagens nach einem der Ansprüche 5 bis 6 verwendet wird, das ein Bindungspeptid nach einem der Ansprüche 1 bis 2 umfasst.

10. Kit zum Anreichern von Mikroorganismen, umfassend:
a) ein Bindungspeptid nach einem der Ansprüche 1 bis 2,
b) einen festen Träger.

11. Kit zum Nachweisen von Mikroorganismen, umfassend:
a) ein Bindungspeptid nach einem der Ansprüche 1 bis 2,
b) einen Marker.

12. Kit zum Nachweisen von *Mycobacterium paratuberculosis,* umfassend:
a) ein Bindungspeptid nach einem der Ansprüche 1 bis 2,
b) eine oder mehrere Nucleinsäuren gemäß SEQ ID NO. 10 und/oder SEQ ID NO. 11.

13. Nucleinsäure mit einem für ein Peptid codierenden Abschnitt, wobei das Peptid eines gemäß einem der Ansprüche 1 und/oder 2 ist.

14. Nucleinsäure nach Anspruch 13, **dadurch gekennzeichnet, dass** die Nucleinsäure einen Ausschnitt mit einer Sequenz gemäß einer der Sequenzen SEQ ID 12 bis SEQ ID 62 umfasst.
